# FASCICULE DE BREVET EUROPEEN

(11) **EP 1 520 534 B1**
(45) Date de publication et mention de la délivrance du brevet: **06.09.2006**
(21) Numéro de dépôt: 04021515.4
(22) Date de dépôt: 10.09.2004
(51) Int. Cl.: A61B 17/16, A61B 17/17, A61F 2/08, B23G 5/06, A61B 17/86

(54) **Intrument destine a la mise en place de vis d'interference dans le domaine de la ligamentoplastie**
Gerät zur Vorbereitung von Knochen für eine Interferenzschraube in der Bandenchirurgie
Instrument for preparation of a bone for an interference screw in ligament surgery

(30) Priorité: 02.10.2003 FR 0311643
(43) Date de publication de la demande: 06.04.2005
(73) Titulaire: Centerpulse France SA, 25460 Etupes Cedex (FR); Desarnaud, Michel, 31400 Toulouse (FR)
(72) Inventeur: Desarnaud, Michel, 25 A, rue Proudhon 25000 Besancon (FR)
(74) Mandataire: Bentz, Jean-Paul

(56) Documents cités:
- FR-A- 2 735 008
- US-A- 5 234 430
- US-A- 5 643 269
- US-A1- 2003 018 337

## Description

La présente invention concerne un instrument destiné à la mise en place de vis d'interférence dans le domaine de la ligamentoplastie.

Un tel instrument est indispensable à la technique de fixation applicable pour la reconstruction de ligaments intra-articulaires mais peut être utilisé également pour la fixation et la reconstruction de tendons et de ligaments extra-articulaires.

Pour cela, il est connu, par le document US 2003/0018337 (le préambule de la revendication 1 est basé sur ce document), un instrument destiné à préparer le logement de la vis d'interférence et du ligament ou greffon dans le but de fixer celui-ci dans la chirurgie de la reconstruction ligamentaire.

Il est donc connu, selon la technique actuelle, de percer un trou ou tunnel du diamètre du ligament ou greffon et à fixer celui-ci par l'insertion de vis d'interférence exerçant une action entre la paroi du tunnel et ledit ligament.

Les vis d'interférence étant en matériau résorbable ou non, plus ou moins fragile, il existe des tarauds adaptés au filetage de la vis pour faciliter l'insertion de celle-ci.

La mise en place de la vis d'interférence dans le tunnel formé par un trou borgne fémoral ou dans un trou borgne tibial est donc délicate.

La difficulté, qui n'est pas résolue par le document US 2003/0018337 précité, tient essentiellement au fait que lors du vissage de la vis d'interférence, celle-ci provoque une rotation par entraînement du ligament ou tendon dans le tunnel qui est inégale, en fonction des diamètres de tous les composants en présence et du côté opéré (fémur droit ou fémur gauche).

En fait, il a été constaté que de manière régulière, le greffon tourne partiellement avec la vis d'interférence ce qui induit une mauvaise position et par la même, une moins bonne isométrie.

Il faut savoir que la précision attendue dans ce type d'opération est de l'ordre du demi millimètre ou du millimètre, mais qu'en cas de rotation du greffon l'erreur de positionnement peut avoisiner 3 à 5 mm.

Selon une première phase de la démarche inventive, il a été recherché un instrument qui permet de calibrer et de positionner les tunnels destinés à recevoir les ligaments ou greffons et de conserver la position de ceux-ci lors des vissages de la vis d'interférence.

La standardisation du diamètre du logement influe directement sur l'efficacité de la fixation.

Selon les techniques opératoires plastiques connues, l'instrument consiste en un taraud qui a pour effet de préparer le futur trajet de la vis d'interférence afin de diminuer le couple de frottement, mais en aucun cas ce taraud connu permet d'éviter les phénomènes de glissement des greffons, provoquant leur rotation, comme évoqué ci-dessus.

La présente invention a pour but de remédier aux différents inconvénients connus dans l'art antérieur et ci-dessus cités.

A cet effet, elle concerne un instrument destiné à la mise en place de vis d'interférence dans le domaine de la ligamentoplastie intra-articulaires ou extra-articulaires, et comportant à son extrémité une partie foreuse apte à la préparation de l'emplacement de la vis d'interférence, caractérisé en ce que la partie latérale de sa partie foreuse, comporte sur au moins l'une de ses génératrices, un évidement longitudinal ouvert vers l'extérieur, formant un guide destiné au perçage dans l'articulation d'un tunnel sensiblement hémicylindrique.

Il est ainsi possible de réaliser à l'identique des tunnels pour le logement d'un ou plusieurs faisceaux fémoral ou pour la partie tibiale de la reconstruction, mais aussi pour toute fixation tendino-osseuse dans tout type d'articulation.

Un tel instrument selon l'invention permet d'éviter, qu'au moment du vissage proprement dit de la vis d'interférence, celle-ci puisse entraîner dans son filetage le ou les ligament(s) ou greffon(s).

Cette description donnée à titre d'exemple non limitatif, fera mieux comprendre comment l'invention peut être réalisée en référence aux dessins annexés sur lesquels :

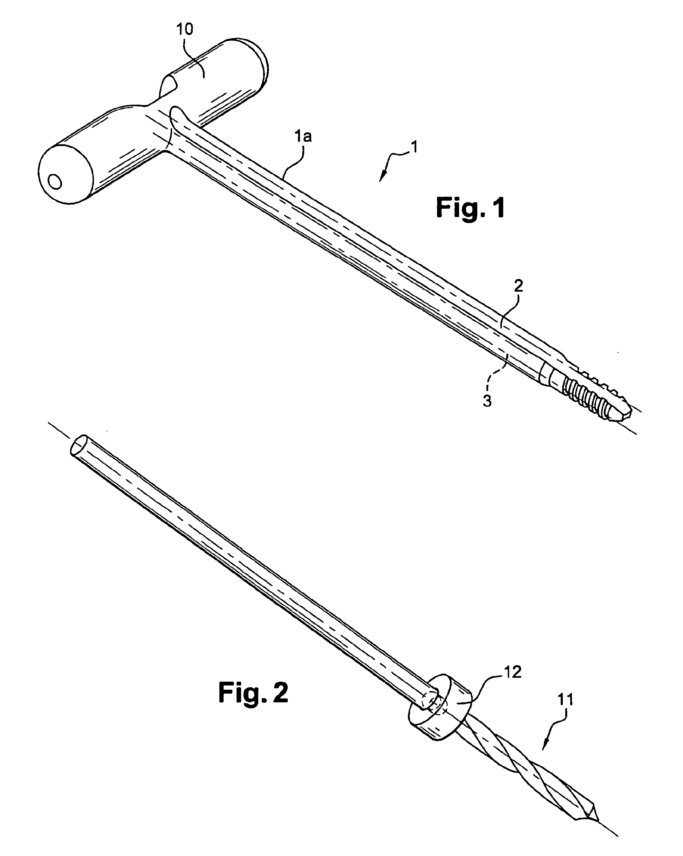

La figure 1 représente en perspective un instrument selon l'invention constitué par l'instrument, en l'occurrence un taraud, pour la préparation d'un trou borgne fémoral ou tibial.
La figure 2 représente en perspective un outil de perçage pour la réalisation de tunnels sécants au trou borgne fémoral ou tibial, préparé par ledit taraud.

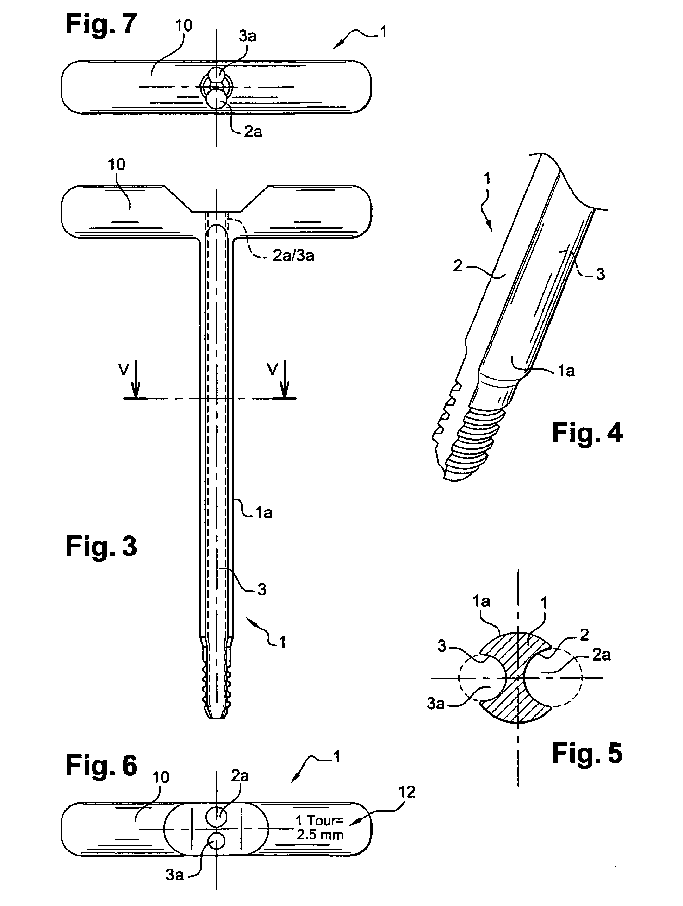

La figure 3 est une vue en plan du taraud selon la figure 1.
La figure 4 est une vue en perspective à échelle agrandie de l'extrémité du taraud, selon les figures 1 et 3.
La figure 5 est une vue en coupe selon la ligne V, V du taraud selon la figure 3.
La figure 6 est une vue de dessus selon la figure 3.
La figure 7 est une vue de dessous selon la figure 3.

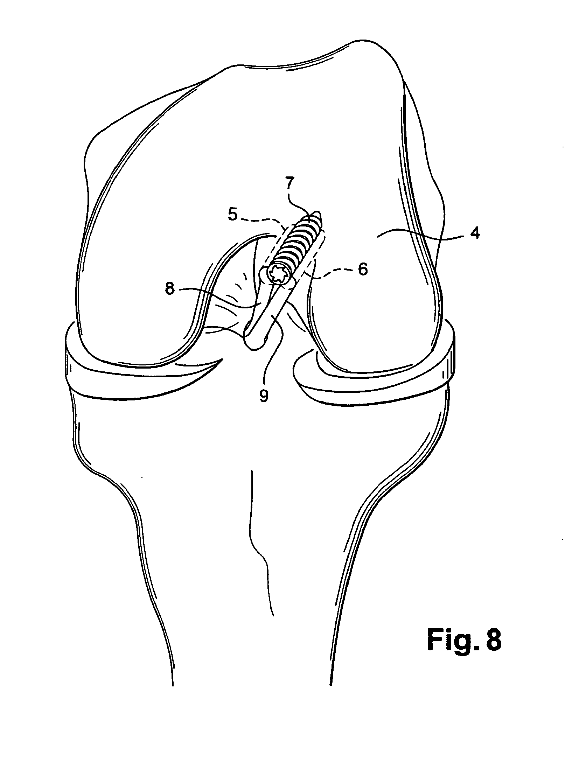

La figure 8 est une vue en perspective d'un genou ayant fait l'objet d'une reconstruction de ligaments intra-articulaires.

L'instrument 1 désigné globalement sur les figures, objet de l'invention, se caractérise en ce que la partie latérale la de sa partie foreuse apte à la préparation de l'emplacement de la vis d'interférence 7, comporte sur au moins l'une de ses génératrices, un évidement longitudinal 2, 3 ouvert vers l'extérieur, formant un guide destiné au perçage dans l'articulation 4 d'un tunnel 5, 6 sensiblement hémicylindrique dont la position en vue d'un perçage sécant avec l'emplacement avec la vis 7 est déterminé par la position horaire dudit instrument 1, de manière à préparer dans un premier temps l'emplacement de la vis d'interférence 7 et dans un deuxième temps, par perçage sécant le tunnel 5, 6 destiné à recevoir un ligament ou greffon 8, 9, afin d'éviter la rotation de ce dernier par entraînement, lors du vissage de la vis d'interférence 7, tout en autorisant l'immobilisation en place dudit ligament 8, 9 par la vis 7.

Selon les présents exemples de réalisation, l'instrument 1 comporte sur une seconde génératrice, diamétralement opposée à la première, un évidement longitudinal 2, 3 ouvert vers l'extérieur, pour former deux guides destinés au perçage de deux tunnels 5, 6 sensiblement hémicylindriques, leur position relative étant déterminée par une première position horaire de l'instrument 1 lors d'un premier perçage et par une deuxième position horaire du même instrument 1 lors d'un second perçage, de manière à déterminer l'emplacement et la réalisation desdits tunnels 5, 6 en vue de la mise en place ultérieure de deux ligaments 8, 9, selon des emplacements choisis et de leur immobilisation à l'aide d'une seule vis d'interférence 7.

Ceci a pour effet de diminuer la quantité de matériaux résorbables ou non implantée, comparée à une technique à deux faisceaux fixés par deux vis d'interférence au niveau fémoral selon l'art antérieur.

En fait, l'espacement entre les tunnels 5, 6 destiné à la mise en place de deux ligaments fémoraux 8, 9 est maîtrisé par l'intermédiaire de l'instrument 1 à deux évidements longitudinaux 2, 3.

C'est la rotation de l'instrument 1 qui défini le bon positionnement des tunnels 5, 6 à réaliser.

L'éloignement de ses tunnels est défini par la position horaire de l'instrument lors des perçages.

Ceux-ci sont à égale distance du centre des vis et la distance par rapport à la cortical est donnée par l'utilisation d'un viseur fémoral décalé à 7, 8 ou 9 mm, connu dans l'art antérieur.

Selon une autre caractéristique de l'invention, la partie foreuse de l'instrument 1 est constitué par un taraud, surmonté par une poignée amovible 10 dans laquelle débouche les évidements 2, 3 sous forme de deux trous 2a, 3a du diamètre des tunnels 5, 6 à réaliser ultérieurement dans l'articulation 4, une telle poignée 10 permettant à la fois l'actionnement en rotation du taraud 1 selon une profondeur prédéterminée et l'engagement d'un outil de forage 11 dans chacun des trous 2a, 3a afin de réaliser lesdits tunnels 5, 6 selon des emplacements espacés angulairement, également de manière prédéterminée.

Ainsi, et comme déjà évoqué les trous 2a, 3a sont réalisés dans la poignée 10 du taraud 1 à égale distance du centre de celui-ci, d'où du centre de la vis d'interférence 7 à mettre en place ultérieurement.

Les diamètres des trous 2a, 3a réalisés dans la poignée 10 du taraud 1 et le diamètre des évidements hémicylindrique 2, 3 qui les prolongent sont de valeurs différentes en fonction du cas à traiter.

Bien entendu, le taraud pourra être de diamètre et de longueur variables, en fonction du cas à traiter.

Par ailleurs, le perçage du tunnel hémicylindrique 5, 6 est réalisé à l'aide d'un outil de forage 11, mèche ou dilatateur, comportant une butée de fin de forage 12 et/ou une graduation.

Egalement, la vis d'interférence 7 est réalisée en un matériau résorbable ou non résorbable.

La partie supérieure de la poignée pourra également comporter des indications 12 indiquant par exemple qu'un tour de taraud correspond à un enfoncement de 2,5mm.

En résumé, le taraud 1 doit permettre, par l'intermédiaire de la poignée 10 percée de deux trous 2a, 3a, se prolongeant sur toute la longueur du taraud 1 par les évidements 2, 3 :
- de visser le taraud 1 dans un trou borgne préalablement réalisé dans l'articulation 4, en creusant le filet de la vis d'interférence 7,
- une fois le taraud 1 introduit dans le condyle, il est possible d'engager un outil de forage pouvant être en butée ou graduée, afin de préparer les tunnels 5, 6 des ligaments 8, 9 correspondants aux faisceaux fémoraux.

Les évidements longitudinaux 2, 3 ainsi que les trous les surmontant 2a, 3a sont percés dans le corps du taraud dans le but d'une ergonomie optimisée.

Il est possible d'avoir une poignée 10 amovible afin de faciliter la mise en place des outils de forage 11.

Il est possible d'avoir des évidements 2, 3 non rétentifs, semi-rétentifs ou complètement rétentifs.

Il est également possible d'avoir un filet de taraud taraudant ou auto-taraudant.

Il est possible également d'utiliser un instrument qui ne soit pas un taraud, en fait, un instrument de perçage tel qu'un foret ou des dilatateurs, tout comme il est possible de réaliser un instrument monobloc incluant un impacteur dilatateur monobloc pour réaliser les trois perçages simultanément à savoir : les deux tunnels 5, 6 ; et le trou borgne constituant l'emplacement de la vis d'interférence 7.

Le taraud 1 tel que décrit ci-dessus peut avoir des diamètres variables avec des trous destinés à des greffons de diamètres différents.

Par exemple, un taraud de diamètre 7mm sera muni de deux trous destinés au logements des greffons de diamètres 4mm - 5mm.

La facilité avec laquelle la vis 7 peut être introduite dans le trou borgne qui lui est destiné peut même amener le chirurgien a changer la taille de la vis 7.

Ainsi, il lui est possible de surdimensionner la vis d'un ou deux millimètres.

Par exemple une vis de 8mm sur un taraudage de 7mm ou une vis de 9mm sur un taraudage à 8mm, ceci pour améliorer la stabilité primaire en fonction de la densité osseuse rencontrée.

## Revendications

1. Instrument (1) destiné à la mise en place de vis d'interférence (7) dans le domaine de la ligamentoplastie intra-articulaires ou extra-articulaires, et comportant à son extrémité une partie foreuse apte à la préparation de l'emplacement de la vis d'interférence (7), **caractérisé en ce que** la partie latérale (1a) de sa partie foreuse, comporte sur au moins l'une de ses génératrices, un évidement longitudinal (2, 3) ouvert vers l'extérieur, formant un guide destiné au perçage, sur la périphérie de l'emplacement destiné à la vis d'interférence, d'un tunnel (5, 6) sensiblement hémicylindrique.

2. Instrument selon la revendication 1, **caractérisé en ce qu'**il comporte sur une seconde génératrice diamétralement opposée à la première, un second évidement longitudinal (3, 2) ouvert vers l'extérieur, pour former un second guide destiné au perçage d'un second tunnel (6, 5) sensiblement hémicylindrique.

3. Instrument selon la revendication 2, **caractérisé en ce que** sa partie foreuse est constituée par un taraud (1) surmonté par une poignée amovible (10) dans laquelle débouche les évidements (2, 3) sous forme de deux trous (2a, 3a) du diamètre des tunnels (5, 6) à réaliser ultérieurement sur la périphérie de l'emplacement destiné à la vis d'interférence, une telle poignée (10) permettant l'actionnement en rotation du taraud (1) et l'engagement d'un outil de forage (11) dans chacun des trous (2a, 3a) afin de réaliser lesdits tunnels (5, 6) selon des emplacements espacés angulairement, également de manière prédéterminée.

4. Instrument selon la revendication 3, **caractérisé en ce que** les trous (2a, 3a) sont réalisés dans la poignée (10) du taraud (1) à égale distance du centre de celui-ci, d'où du centre de la vis d'interférence (7) à mettre en place ultérieurement.

5. Instrument selon l'une des revendications 3 ou 4, **caractérisé en ce que** le diamètre des trous (2a, 3a) réalisés dans la poignée (10) du taraud (1) et le diamètre des évidements hémicylindrique (2, 3) qui les prolongent sont de valeurs différentes en fonction du cas à traiter.

6. Instrument selon l'une des revendications 3 à 5, **caractérisé en ce que** le taraud (1) qui le constitue est de diamètre et de longueur différentes en fonction du cas à traiter.

## Claims

1. Instrument (1) for placement of interference screws (7) in intra -articular or extra -articular ligament surgery, comprising, at its end, a drill part for preparing the site for the interference screw (7), **characterized in that** the lateral part (1a) of its drill part has, on at least one of its generatrices, a longitudinal recess (2, 3) opening towards the outside and forming a guide for boring a substantially semicylindrical tunnel (5, 6) on the periphery of the site intended for the interference screw.

2. Instrument according to Claim 1, **characterized in that** it comprises , on a second generatrix diametrically opposite to the first one, a second longitudinal recess (3, 2) opening towards the outside in order to form a second guide for boring a second substantially semicylindrical tunnel (6, 5) .

3. Instrument according to Claim 2, **characterized in that** its drill part is composed of a screw tap (1) surmounted by a movable handle (10) in which the recesses (2, 3) open out in the form of two ho les (2a, 3a) with the diameter of the tunnels (5, 6) that are to be produced subsequently on the periphery of the site intended for the interference screw, such a handle (10) permitting rotation of the screw tap (1) and engagement of a drilling tool (11) in each of the holes (2a, 3a) so as to produce said tunnels (5, 6) at sites that are spaced apart angularly, also in a predetermined manner.

4. Instrument according to Claim 3, **characterized in that** the holes (2a, 3a) are formed in the handle (10) of the s crew tap (1) at an equal distance from the centre of the latter, hence from the centre of the interference screw (7) to be subsequently put in place.

5. Instrument according to one of Claims 3 and 4, **characterized in that** the diameter of the holes (2a, 3a) formed in the handle (10) of the screw tap (1) and the diameter of the semicylindrical recesses (2, 3) which continue them are of different values depending on the case that is to be treated.

6. Instrument according to one of Claims 3 to 5, **characterized in that** the screw tap (1) of which it is composed has a different diameter and length depending on the case that is to be treated.

## Patentansprüche

1. Instrument (1) für den Einbau einer Interferenzschraube (7) im Bereich der intraartikulären oder extraartikulären Bandplastik, welches Instrument an seinem Ende einen Bohrabschnitt zur Vorbereitung des Einbauplatzes der Interferenzschraube (7) aufweist, **dadurch gekennzeichnet, daß** der laterale Teil (1a) des Bohrabschnitts auf mindestens einer seiner Umfangslinien eine nach außen offene Längsaussparung (2, 3) aufweist, die eine Führung zum Bohren eines im wesentlichen halbzylindrischen Tunnels (5, 6) am Rand des Einbauplatzes für die Interferenzschraube (7) bildet.

2. Instrument nach Anspruch 1, **dadurch gekennzeichnet, daß** es auf einer zweiten Umfangslinie diametral gegenüberliegend von der Ersten eine zweite longitudinale, nach außen offene Längsaussparung (3, 2) aufweist, um eine zweite Führung zum Bohren eines zweiten im wesentlichen halbzylindrischen Tunnels (6, 5) zu bilden.

3. Instrument nach Anspruch 2, **dadurch gekennzeichnet, daß** sein Bohrabschnitt durch einen Gewindebohrer (1) gebildet ist, der einen abnehmbaren Griff (10) trägt, in dem die Aussparungen (2, 3) in Form von zwei Löchern (2a, 3a) des Durchmessers der Tunnel (5, 6) münden, die später am Rand des Einbauplatzes für die Interferenzschraube erstellt werden, wobei der Griff (10) eine Drehbetätigung des Gewindebohrers (1) und den Eingriff eines Bohrwerkzeuges (11) in jedem Loch (2a, 3a) ermöglicht, um die Tunnel (5, 6) gemäß der in gleichfalls vorbestimmter Art winklig voneinander beabstandeten Einbauplätze zu erstellen.

4. Instrument nach Anspruch 3, **dadurch gekennzeichnet, daß** die Löcher (2a, 3a) in dem Griff (10) des Gewindebohrers (1) in gleichem Abstand zur Mitte desselben erstellt sind, also zur Mitte der Interferenzschraube (7), die später einzubauen ist.

5. Instrument nach Anspruch 3 oder 4, **dadurch gekennzeichnet, daß** der Durchmesser der Löcher (2a, 3a), die in dem Griff (10) des Gewindebohrers (1) erstellt sind, und der Durchmesser der halbzylindrischen Aussparungen (2, 3), die sie verlängern, in Abhängigkeit des zu behandelnden Falls verschiedene Größen aufweisen.

6. Instrument nach einem der Ansprüche 3 bis 5, **dadurch gekennzeichnet, daß** der Gewindebohrer (1), der es bildet, verschiedene Durchmesser und Längen in Abhängigkeit des zu behandelnden Falls aufweist.
